# FASCICULE DE BREVET EUROPEEN

(11) **EP 0 911 051 B1**
(45) Date de publication et mention de la délivrance du brevet: **11.06.2003**
(21) Numéro de dépôt: 98402655.9
(22) Date de dépôt: 26.10.1998
(51) Int. Cl.: A61M 16/12

(54) **Dispositif d'assistance respiratoire**
Vorrichtung zur Unterstützung der Atmung
Respiratory assistance device

(30) Priorité: 27.10.1997 FR 9713430
(43) Date de publication de la demande: 28.04.1999
(73) Titulaire: Boussignac, Georges, 92160 Antony (FR); Labrune, Jean-Claude, F-92310 Sèvres (FR)
(72) Inventeur: Boussignac, Georges, 92160 Antony (FR); Labrune, Jean-Claude, F-92310 Sèvres (FR)
(74) Mandataire: Bonnetat, Christian

(56) Documents cités:
- EP-A- 0 390 684
- EP-A- 0 640 355
- EP-A- 0 701 834
- WO-A-97/06843
- DE-U- 9 202 198
- GB-A- 2 267 661
- US-A- 4 558 708
- US-A- 5 474 060

## Description

La présente invention a pour objet un dispositif d'assistance respiratoire, utilisable sur des patients dont la respiration spontanée est absente ou insuffisante, qu'ils soient ou non placés sous respiration artificielle.

Par le brevet européen EP-A-0 390 684 (US-A-5 036 847), on connaît déjà un dispositif d'assistance respiratoire tubulaire, qui forme un canal principal et qui est destiné à être relié par son extrémité distale à une voie respiratoire d'un patient pour que ledit canal principal relie à l'extérieur le système respiratoire dudit patient, ledit dispositif comportant au moins un canal auxiliaire associé à des moyens de déflexion pour injecter un jet de gaz respiratoire défléchi vers l'intérieur dudit canal principal et destiné à la ventilation dudit patient.

La présente invention a pour objet de perfectionner un tel dispositif afin que le praticien puisse connaître la pression gazeuse et/ou la composition gazeuse dans ladite voie respiratoire du patient.

A cette fin, selon l'invention, le dispositif d'assistance respiratoire du type rappelé ci-dessus, est remarquable en ce qu'il comporte, du côté distal, une chambre annulaire, disposée à la périphérie dudit dispositif tubulaire, coaxialement à celui-ci et adaptée pour la communication avec le système respiratoire du patient par l'intermédiaire de l'orifice formé par sa section annulaire distale, et en ce que ladite chambre annulaire est pourvue de moyens de liaison avec l'extérieur.

Ainsi, il est possible de mesurer la pression et/ou de déterminer la composition du gaz se trouvant dans ladite chambre. On remarquera que ce gaz est identique à celui régnant dans le système respiratoire, mais est assagi par rapport à celui-ci, du fait de son isolation partielle par la chambre. Les mesures sont donc fiables, en évitant les conséquences des turbulences gazeuses.

Notamment à des fins d'homogénéisation du gaz contenu dans la chambre, il est alors préférable que lesdits moyens de liaison avec l'extérieur soient disposés à l'extrémité proximale de ladite chambre.

Dans le cas où les gaz se trouvant dans ladite chambre doivent faire l'objet d'une analyse de composition et d'une mesure de pression, ladite chambre doit présenter un volume relativement grand, correspondant à la quantité de gaz nécessaire à l'analyse. Il peut alors se produire, dans ladite chambre, des turbulences susceptibles d'induire de fortes variations dans les mesures de pression.

Pour éviter cela, il est avantageux de disposer, dans ladite chambre, des moyens d'amortissement de turbulences gazeuses, tels qu'un filtre fibreux ou poreux.

A titre de sécurité supplémentaire à un éventuel dispositif de mesure de pression, il est avantageux que le dispositif conforme à la présente invention comporte un réseau de rainures pratiquées dans la paroi du canal principal et au moins un orifice traversant la partie proximale dudit dispositif, de façon à former une mise à l'air libre du système respiratoire dudit patient, en cas de bouchage accidentel de la partie proximale dudit dispositif.

Le dispositif d'assistance respiratoire tubulaire conforme à la présente invention peut trouver de nombreuses applications. Il peut être utilisé comme sonde buccale ou nasale, ou bien encore comme embout rapporté à un tube de trachéotomie. Il peut également, dans un masque respiratoire destiné à être appliqué sur le visage d'un patient, constituer l'embout tubulaire d'entrée et de sortie du gaz respiratoire. Dans ce cas, ladite chambre est en communication avec le système respiratoire du patient par l'intermédiaire de l'intérieur dudit masque.

Lorsqu'il est utilisé comme embout de masque respiratoire, le dispositif d'assistance respiratoire conforme à la présente invention peut être solidaire dudit masque. Cependant, notamment pour pouvoir être utilisé seul, sans masque, il peut être avantageux que ledit dispositif soit monté de façon amovible, par exemple par emboîtement, sur ledit masque.

Les figures du dessin annexé feront bien comprendre comment l'invention peut être réalisée. Sur ces figures, des références identiques désignent des éléments semblables.
La figure 1 est une vue schématique, partiellement en coupe axiale, d'un masque d'assistance respiratoire comportant un dispositif tubulaire conforme à la présente invention.
La figure 2 montre, en coupe axiale, ledit dispositif tubulaire constituant l'embout du masque de la figure 1.
Les figures 3, 4 et 5 sont des coupes transversales du dispositif de la figure 2, respectivement selon les lignes III-III, IV-IV et V-V.
La figure 6 montre, en coupe axiale semblable à la figure 2, une variante de réalisation du dispositif tubulaire conforme à la présente invention.
Les figures 7, 8 et 9 sont des coupes transversales du dispositif de la figure 6, respectivement selon les lignes VII-VII, VIII-VIII et IX-IX.

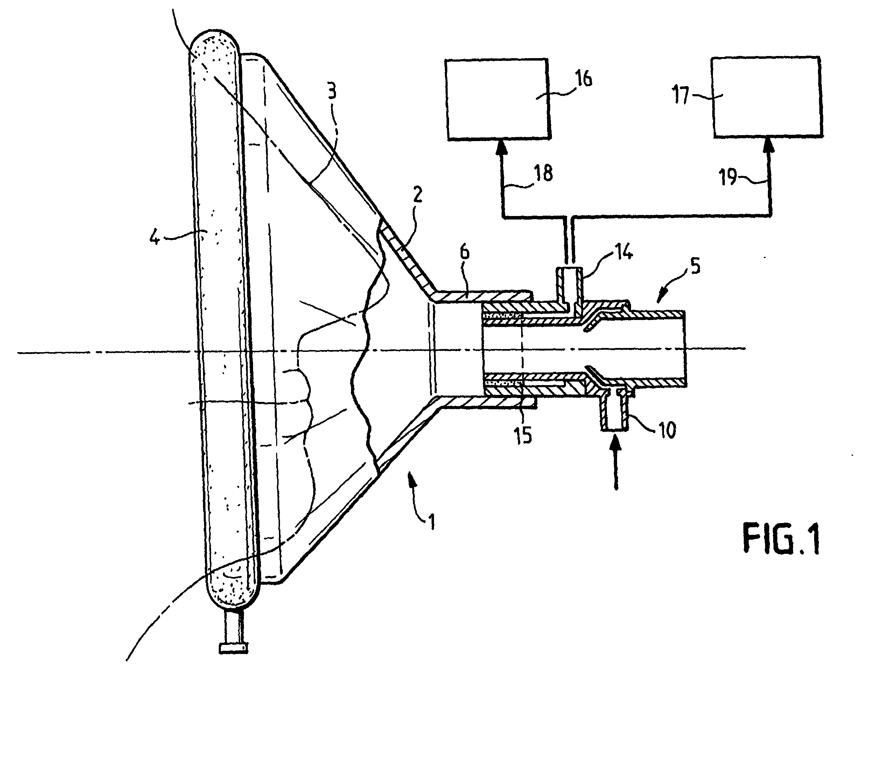

Le masque d'assistance respiratoire 1, conforme à la présente invention et représenté sur la figure 1, comporte une coque rigide de forme générale tronconique 2, pouvant être appliquée sur le visage 3 d'un patient par l'intermédiaire d'un coussinet gonflable 4, bordant son ouverture périphérique. Du côté opposé, ledit masque 1 comporte un dispositif tubulaire 5, fixé ou emboîté sur une saillie tubulaire 6 de ladite coque 2. Le dispositif tubulaire 5 sert d'embout d'entrée et de sortie de gaz respiratoire dans le masque 1. Eventuellement, il peut être relié à un appareil de respiration artificielle par son extrémité proximale (opposée au patient 3).

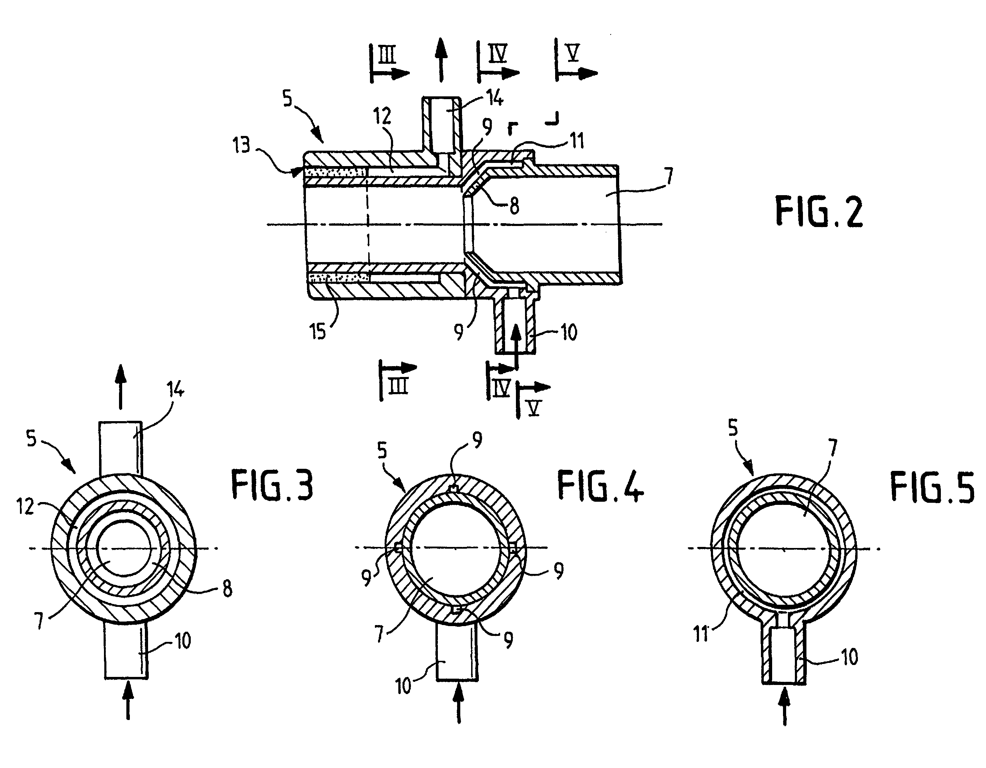

Comme le montrent plus en détail les figures 2 à 5, le dispositif tubulaire 5 comporte un passage traversant interne 7 et, en partie médiane, une paroi conique 8, saillant à l'intérieur dudit passage 7. La paroi conique 8 a pour objet de défléchir, en direction de l'axe du passage 7, des jets de gaz respiratoire injectés par des canaux périphériques 9, alimentés à partir d'un embout d'amenée 10, par l'intermédiaire d'une chambre annulaire périphérique 11.

Par ailleurs, du côté distal, le dispositif tubulaire 5 comporte une chambre périphérique annulaire 12, coaxiale audit dispositif 5. La chambre périphérique annulaire 12 communique avec l'intérieur du masque 1 par son orifice annulaire distal 13 et est pourvue, à son extrémité proximale, d'un embout de sortie 14.

Un filtre 15, fibreux ou poreux (coton, mousse synthétique, etc...) est disposé dans la chambre périphérique annulaire 12, pour amortir les turbulences gazeuses et, par suite, les trop fortes variations de pression.

Comme cela est représenté sur la figure 1, l'embout de sortie 14 peut être relié à un analyseur de gaz 16 et à un dispositif de mesure de pression 17. Bien entendu, les liaisons 18 et 19 entre l'embout de sortie 14 et les dispositifs 16 et 17 sont prévues pour que le prélèvement de gaz à analyser par la liaison 18 n'ait pas d'influence sur la mesure de pression, via la liaison 19.

Ainsi, grâce aux dispositifs 16 et 17, le praticien assistant le patient 3 connaît en permanence la composition du gaz dans le masque 1 (et notamment sa teneur en gaz carbonique) et la pression à l'intérieur dudit masque. Il peut donc prendre les mesures d'intervention appropriées en fonction desdites composition et pression du gaz.

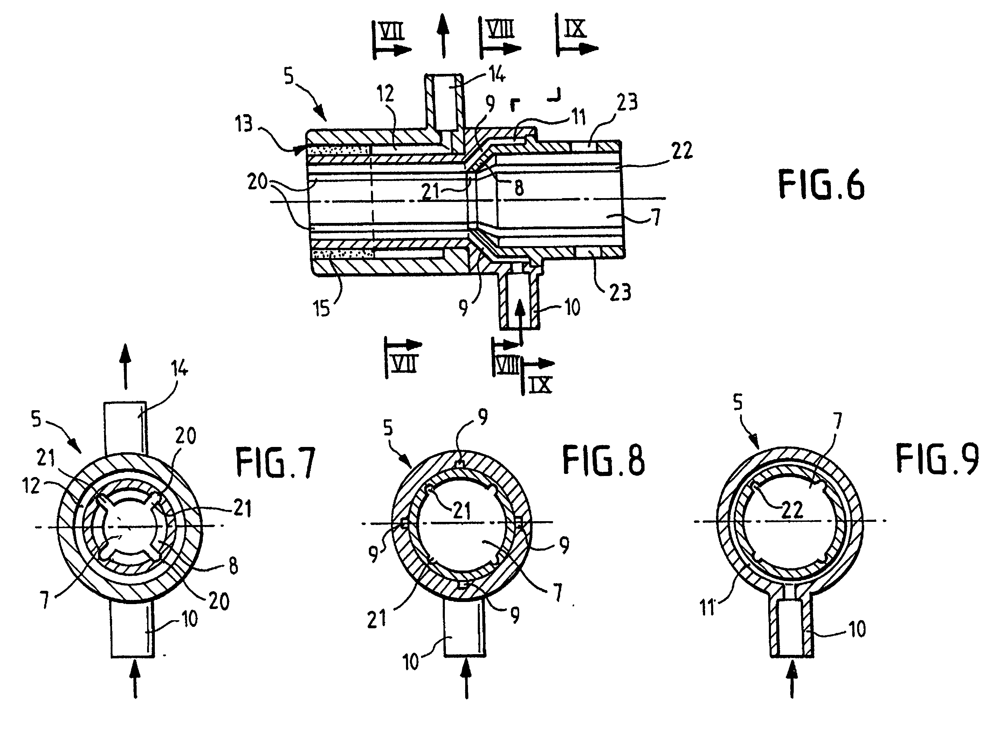

Dans la variante de réalisation du dispositif 5, montrée par les figures 6 à 9, on retrouve les éléments 7 à 15 précédemment décrits en regard des figures 2 à 5. Cependant, on peut remarquer que cette variante de réalisation comporte de plus, dans la paroi du passage 7 et dans la paroi conique 8, des rainures 20, 21, 22 disposées radialement entre les canaux périphériques 9, ainsi qu'au moins un orifice 23 traversant la paroi de l'extrémité proximale du dispositif 5. Ainsi, si pour une raison quelconque la partie proximale du dispositif 5 est bouchée, les rainures 20, 21, 22 et les orifices 23 constituent une mise à l'air libre évitant à la pression du gaz respiratoire injecté d'atteindre des valeurs excessives, susceptibles d'être préjudiciables au patient. On obtient ainsi une sécurité de fonctionnement automatique, complétant l'éventuel dispositif de mesure de pression 17.

Bien que sur les figures, on ne les ait pas respectés, il va de soi que le dispositif 5 peut comporter des canaux ou conduits d'injection de médicaments et/ou d'eau.

Par ailleurs, bien que ci-dessus on ait décrit, à l'aide desdites figures, une application particulière du dispositif 5 à un masque respiratoire, on comprendra aisément que ledit dispositif peut trouver de nombreuses autres applications, par exemple comme sonde nasale, sonde buccale, sonde trachéale, etc ... Bien entendu, les dimensions dudit dispositif sont adaptées à chaque utilisation particulière.

## Revendications

1. Dispositif d'assistance respiratoire tubulaire (5), qui forme un canal principal (7) et qui est destiné à être relié par son extrémité distale à une voie respiratoire d'un patient pour que ledit canal principal (7) relie à l'extérieur le système respiratoire dudit patient, ledit dispositif comportant au moins un canal auxiliaire (9) associé à des moyens de déflexion (8) pour injecter un jet de gaz respiratoire défléchi vers l'intérieur dudit canal principal (7) et destiné à la ventilation dudit patient,
**caractérisé en ce qu'**il comporte, du côté distal, une chambre annulaire (12), disposée à la périphérie dudit dispositif tubulaire (5), coaxialement à celui-ci et adaptée pour la communication avec le système respiratoire du patient par l'intermédiaire de l'orifice (13) formé par sa section annulaire distale, et **en ce que** ladite chambre annulaire (12) est pourvue de moyens (14) de liaison avec l'extérieur.

2. Dispositif d'assistance respiratoire selon la revendication 1,
**caractérisé en ce que** lesdits moyens de liaison (14) sont disposés à l'extrémité proximale de ladite chambre (12).

3. Dispositif d'assistance respiratoire selon l'une des revendications 1 ou 2,
**caractérisé en ce que** les gaz contenus dans ladite chambre font l'objet d'une analyse de composition et d'une mesure de pression, et **en ce que** des moyens (15) d'amortissement de turbulences gazeuses sont disposés dans ladite chambre (12).

4. Dispositif d'assistance respiratoire selon la revendication 3,
**caractérisé en ce que** lesdits moyens d'amortissement des turbulences gazeuses sont formés par un filtre d'une matière poreuse.

5. Dispositif d'assistance respiratoire selon la revendication 3,
**caractérisé en ce que** lesdits moyens d'amortissement des turbulences gazeuses sont formés par un filtre annulaire d'une matière fibreuse.

6. Dispositif d'assistance respiratoire selon l'une quelconque des revendications 1 à 5,
**caractérisé en ce qu'**il comporte un réseau de rainures (20, 21, 22) pratiquées dans la paroi dudit canal principal (7) et au moins un orifice (23) traversant la partie proximale dudit dispositif (5), de façon à former une mise à l'air libre du système respiratoire dudit patient, en cas de bouchage accidentel de la partie proximale dudit dispositif (5).

7. Dispositif d'assistance respiratoire selon l'une quelconque des revendications 1 à 6,
**caractérisé en ce qu'**il constitue l'embout d'entrée et de sortie de gaz respiratoire d'un masque (1) destiné à être appliqué sur le visage dudit patient.

8. Dispositif d'assistance respiratoire selon la revendication 7,
**caractérisé en ce qu'**il est solidaire dudit masque (1).

9. Dispositif d'assistance respiratoire selon la revendication 7,
**caractérisé en ce qu'**il est monté de façon amovible sur ledit masque (1).

10. Dispositif d'assistance respiratoire selon la revendication 9,
**caractérisé en ce qu'**il est emboîtable sur ledit masque (1).

## Claims

1. Tubular respiratory assistance device (5), which forms a main channel (7) and which is intended to be connected by its distal end to a patient's airway so that the said main channel (7) connects the said patient's respiratory system to the outside, the said device having at least one auxiliary channel (9) associated with deflection means (8) for injecting a respiratory gas jet which is deflected towards the interior of the said main channel (7) and is intended to ventilate the said patient, **characterized in that** it has, on the distal side, an annular chamber (12) arranged at the periphery of the said tubular device (5), coaxially with it and suitable for communicating with the patient's respiratory system by means of the orifice (13) formed by its distal annular section, and **in that** the said annular chamber (12) is provided with means (14) for connection to the outside.

2. Respiratory assistance device according to Claim 1, **characterized in that** the said connecting means (14) are arranged at the proximal end of the said chamber (12).

3. Respiratory assistance device according to one of Claims 1 and 2, **characterized in that** the gases contained in the said chamber undergo compositional analysis and pressure measurement, and **in that** means (15) for damping gas turbulence are arranged in the said chamber (12).

4. Respiratory assistance device according to Claim 3, **characterized in that** the said means for damping gas turbulence are formed by a filter of a porous material.

5. Respiratory assistance device according to Claim 3, **characterized in that** the said means for damping gas turbulence are formed by an annular filter of a fibrous material.

6. Respiratory assistance device according to any one of Claims 1 to 5, **characterized in that** it has a network of grooves (20, 21, 22) made in the wall of the said main channel (7) and at least one orifice (23) passing through the proximal part of the said device (5) so as to form an access to the atmosphere for the said patient's respiratory system, in the event of accidental obstruction of the proximal part of the said device (5).

7. Respiratory assistance device according to any one of Claims 1 to 6, **characterized in that** it constitutes the adapter for inlet and outlet of respiratory gas of a mask (1) intended to be fitted to the said patient's face.

8. Respiratory assistance device according to Claim 7, **characterized in that** it is integral with the said mask (1).

9. Respiratory assistance device according to Claim 7, **characterized in** it is fitted removably to the said mask (1).

10. Respiratory assistance device according to Claim 9, **characterized in that** it can be engaged on the said mask (1).

## Patentansprüche

1. Rohrförmige Vorrichtung zur Unterstützung der Atmung (5), welche einen Hauptkanal (7) bildet und dazu bestimmt ist, mit ihrem distalen Ende mit einem Atemweg eines Patienten dergestalt verbunden zu werden, dass der besagte Hauptkanal (7) die Verbindung des Atemsystems des Patienten nach außen herstellt, wobei die besagte Vorrichtung mindestens einen Hilfskanal (9) enthält, der mit Ablenkmitteln (8) verbunden ist, um einen Strahl von Atemgas einzuleiten, der zum Inneren des besagten Hauptkanals (7) hin abgelenkt wird und der zur Beatmung des besagten Patienten bestimmt ist, **dadurch gekennzeichnet, dass** sie auf der distalen Seite eine Ringkammer (12) aufweist, die auf dem Umfang der besagten rohrförmigen Vorrichtung (5) koaxial mit derselben angeordnet ist und für die Verbindung mit dem Atemsystem des Patienten über eine durch ihren distalen ringförmigen Querschnitt gebildete Öffnung (13) ausgelegt ist, und dadurch, dass die besagte Ringkammer (12) mit Mitteln (14) zur Verbindung nach außen ausgestattet ist.

2. Vorrichtung zur Unterstützung der Atmung nach Anspruch 1, **dadurch gekennzeichnet, dass** die besagten Mittel zur Verbindung (14) am proximalen Ende der besagten Kammer (12) angeordnet sind.

3. Vorrichtung zur Unterstützung der Atmung nach einem der Ansprüche 1 oder 2, **dadurch gekennzeichnet, dass** die in der besagten Kammer enthaltenen Gase Gegenstand einer Analyse auf ihre Zusammensetzung und einer Druckmessung sind und dadurch, dass Mittel (15) zur Dämpfung der Gasturbulenzen in der besagten Kammer (12) vorhanden sind.

4. Vorrichtung zur Unterstützung der Atmung nach Anspruch 3 **dadurch gekennzeichnet, dass** die besagten Mittel zur Dämpfung der Gasturbulenzen aus einem Filter aus einem porösen Material gebildet werden.

5. Vorrichtung zur Unterstützung der Atmung nach Anspruch 3, **dadurch gekennzeichnet, dass** die besagten Mittel zur Dämpfung der Gasturbulenzen aus einem ringförmigen Filter aus einem Fasermaterial gebildet werden.

6. Vorrichtung zur Unterstützung der Atmung nach einem der Ansprüche 1 bis 5, **dadurch gekennzeichnet, dass** sie ein Netz von in der Wandung des besagten Hauptkanals (7) ausgebildeten Rillen (20, 21, 22) und mindestens eine den proximalen Teil der besagten Vorrichtung (5) durchquerende Öffnung (23) dergestalt aufweist, dass ein Zugang des Atemsystems des besagten Patienten zur Außenluft im Fall des zufälligen Verstopfens des proximalen Teils der besagten Vorrichtung (5) gebildet wird.

7. Vorrichtung zur Unterstützung der Atmung nach einem der Ansprüche 1 bis 6, **dadurch gekennzeichnet, dass** sie für das Atemgas das Eingangsstück und das Ausgangsstück einer Maske (1) bildet, die dazu bestimmt ist, an das Gesicht des besagten Patienten angelegt zu werden.

8. Vorrichtung zur Unterstützung der Atmung nach Anspruch 7 **dadurch gekennzeichnet, dass** sie mit der besagten Maske (1) aus einem Stück ist.

9. Vorrichtung zur Unterstützung der Atmung nach Anspruch 7 **dadurch gekennzeichnet, dass** sie auf entfernbare Weise auf die besagte Maske (1) montiert ist.

10. Vorrichtung zur Unterstützung der Atmung nach Anspruch 9, **dadurch gekennzeichnet, dass** sie auf die besagte Maske (1) aufgesetzt werden kann.
